# EUROPEAN PATENT SPECIFICATION

(11) **EP 0 482 633 B1**
(45) Date of publication and mention of the grant of the patent: **25.05.1994**
(21) Application number: 91118146.9
(22) Date of filing: 24.10.1991
(51) Int. Cl.: F16K 5/04, F16K 7/06

(54) **Clamp device**
Absperrvorrichtung
Dispositif d'arrêt

(30) Priority: 25.10.1990 JP 285833/90
(43) Date of publication of application: 29.04.1992
(73) Proprietor: TERUMO KABUSHIKI KAISHA, Tokyo 151 (JP)
(72) Inventor: Obana, Hiroyuki, c/o Terumo Kabushiki Kaisha, Fujinomiya-shi, Shizuoka-ken (JP)
(74) Representative: Casalonga, Axel

(56) References cited:
- US-A- 3 102 710
- US-A- 3 215 394
- US-A- 3 289 999
- US-A- 3 773 290
- US-A- 3 920 215
- US-A- 4 044 989

## Description

The present invention relates to a clamp device which is attached to a soft tube on the way thereof to allow and stop the flow of fluid such as blood and medicine through the tube. The soft tube can be applied to liquid medicine transfusion, blood transfusion, and can be employed as the blood circuit of an artificial lung, the blood circuit of an artificial kidney, and the like.

One of the conventional clamp devices of this type is disclosed in a Published Examined Japanese Utility Model Application No. 51-45911.

In the case of this conventional clamp device, a rotary cam is incorporated into a holder, a manual operation wheel is attached to the rotary cam and the tube passed through a passage of the holder is gradually pressed and deformed by a large-diameter portion of the rotary cam to stop fluid from flowing through the tube.

In the case of this conventional clamp device, however, the supported portion of the rotary cam is located outside the passage of the holder and the following problems are caused accordingly.
1) It is difficult to make the whole of the clamp device compact.
2) The pressing force gradually added to the front surface of the tube by the large-diameter portion of the rotary cam is stronger in the axial direction of the tube than in a direction perpendicular to the axis of the tube. When the pressing force is added to the tube in this manner, the front surface of the tube is pulled in the axial direction of the tube while keeping the back surface thereof held by the inner face of the passage of the holder. Therefore, the force needed to close the tube becomes unnecessarily large and the tube closing capacity of the clamp device is low.
3) The manual operation wheel is usually gripped by one hand and operated by the thumb of this hand. The clamp device can be therefore used only when it has such a space around it that can be occupied by one hand to grip its manual operation wheel, and when it is located adjacent to another equipment, therefore, its operation becomes difficult.

US-A-3 215 394 discloses a clamp device comprising a holder, a rotator and a tube closing means as well as stopper means. The stopper means comprise lugs and pins. The holder is a tunnel-like member which at the same time serves as a guide for the tube.

An object of the present invention is to provide a clamp device more compact in size and capable of closing the tube with a smaller force but with a higher reliability.

Another object of the present invention is to provide a clamp device capable of being positioned nearer the other tool.

According to the present invention, there can be provided a clamp device comprising a holder having a passage through which a tube is passed in a first direction. A rotator is attached to the holder to be rotatable round its rotating axis which extends in a second direction. Bearing means are formed on the inner face of the holder and supported means are formed on the rotator and supported by the bearing means of the holder. Further the clamp device comprises tube closing means arranged at the rotator and positioned eccentric to the rotating axis of the rotator, said tube closing means being moved between a first position where the tube is pressed against the inner face of the passage by the tube closing means and a second position where the tube is released from the tube closing means when the rotator is rotated. In addition a rotary operation lever is attached to the rotator, and stopper means are provided for stopping the rotator relative to the holder at the first and second positions. In this clamp device the line extending along the rotating axis of the rotator is positioned to cross the passage.

According to a desirable aspect of the present invention, a guide sleeve by which part of the passage is defined is formed on the holder, the tube in it is connected to a container and it includes a means for fixing the holder to the container.

According to the present invention, a tube closing section of the rotator presses the tube against the inner face of the passage in the holder to close the tube when the rotator is switched from the second or opening position to the first or closing position by the rotary operation lever under a condition that the tube is passed through the passage in the holder.

The present invention can achieve the following merits in this case.
1) The line extending from at least part of the supported means along the rotating axis of the rotator is positioned to cross the passage. This enables the whole of the clamp device to be smaller-sized.
2) The line extending from at least part of the supported means along the rotating axis of the rotator is positioned to cross the passage. Therefore, the pressing force added to the surface of the tube by the tube closing section of the rotator is stronger in a direction perpendicular to the axis of the tube. The tube can be thus more effectively closed as if it were pressed and cut by the tube closing section of the rotator in the direction perpendicular to the axis of the tube. As the result, it can be closed with a smaller force but with a higher reliability.
3) The fixing guide sleeve of the holder is fitted onto an outlet port of the container together with the tube passed through the passage. The clamp device can be thus supported by the container and the rotary operation lever can be rotated by fingers of one hand without gripping the holder by hand. No space is therefore needed around the clamp device to grip the holder by hand and this makes it possible to locate the clamp device nearer another equipment such as the container.

When the center axis of the rotator is positioned to cross the passage in the inside thereof in the case of the present invention, the above-mentioned merits can be by far more reliably realized.

This invention can be more fully understood from the following detailed description when taken in conjunction with the accompanying drawings, in which:
Fig. 1 is a plan view showing the clamp device according to a first embodiment of the present invention;
Fig. 2 is a front view showing the clamp device;
Fig. 3A is a plan view showing the clamp device dismantled;
Fig. 3B is a side view showing the clamp device dismantled;
Fig. 4 is a sectional view taken along a line IV - IV in Fig. 1;
Figs. 5A and 5B are front and sectional views, respectively, showing the clamp device kept under opening mode;
Figs. 6A and 6B are front and sectional views, respectively, showing the clamp device kept under closing mode;
Fig. 7 is a sectional view showing the main portion of the clamp device according to a second embodiment of the present invention;
Fig. 8 is a sectional view showing the main portion of the clamp device according to a third embodiment of the present invention;
Figs. 9A and 9B are sectional views showing a holder and rotator, respectively, for use with the clamp device according to a fourth embodiment of the present invention; and
Fig. 10 schematically shows the clamp device in Fig. 1 kept under use.

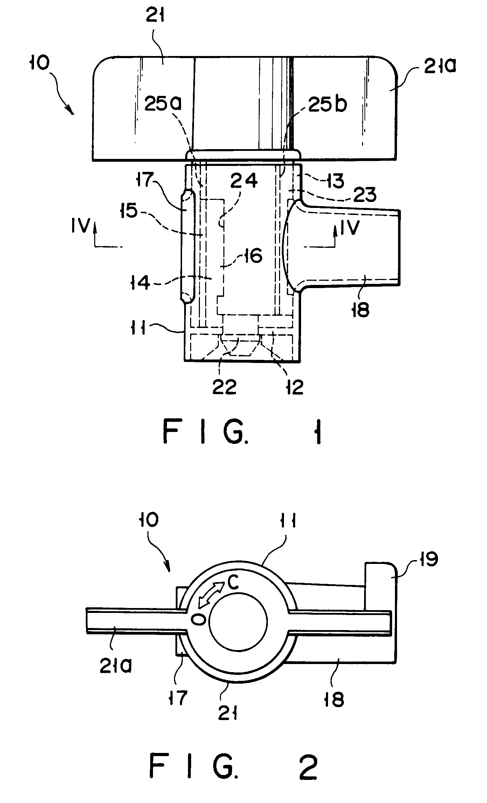

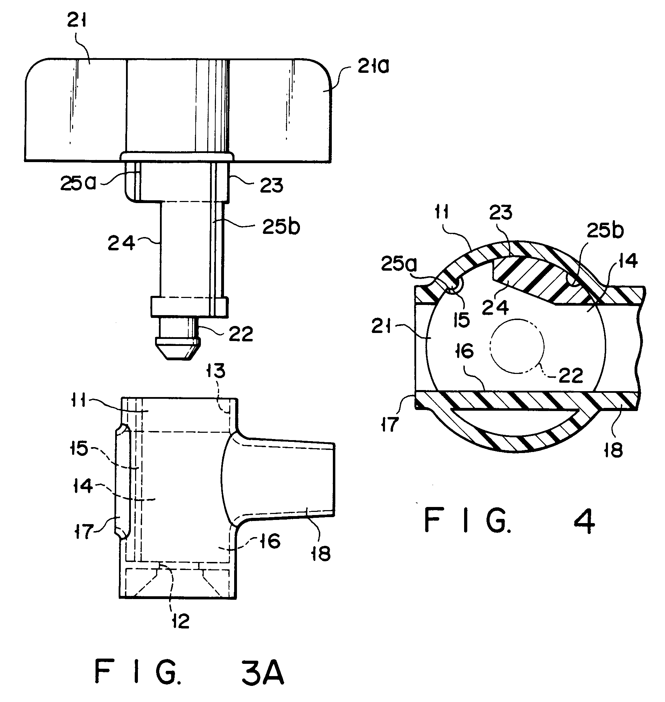

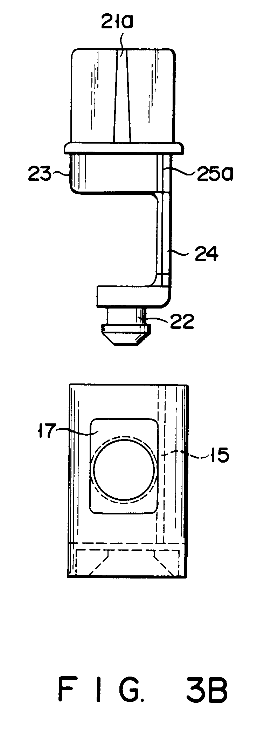

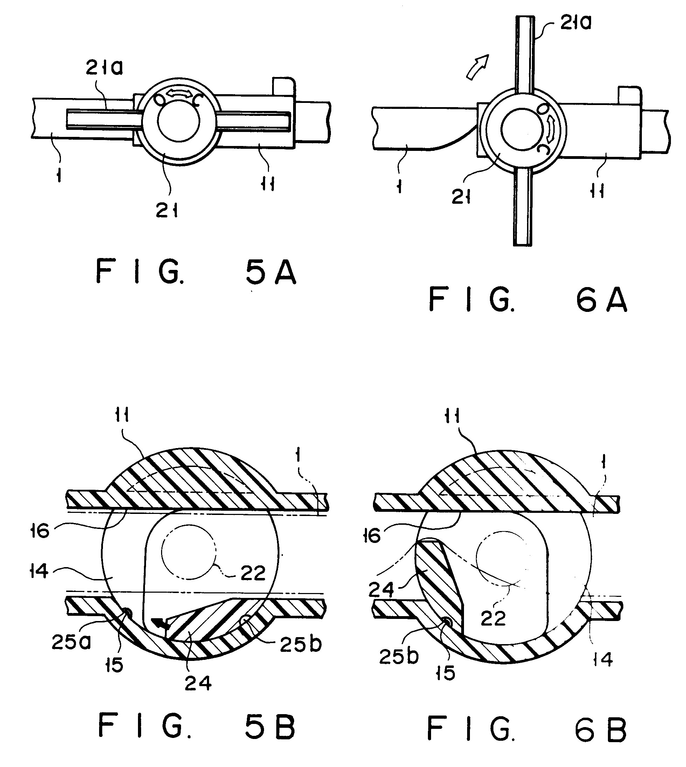

As shown in Figs. 1 through 4, a clamp device 10 comprises a holder 11 and a rotator 21 incorporated into the holder 11, and it is intended to open and close a resin-made soft tube 1 (shown in Figs. 5A, 5B, 6A and 6B) to allow and stop the flow of fluid such as a liquid medicine or a humor (blood) through the tube 1. The holder 11 is shaped substantially like a cylinder, having small- and large-diameter bearing sections 12 and 13 at both ends thereof to hold the rotator 21, a passage 14 at the center thereof to allow the tube 1 to be passed therethrough, and a projected stopper 15 on the inner circumference thereof to tightly stop the rotator 21 at those positions where the tube 1 is opened and closed. That inner face of the passage 14 which faces the rotator 21 serves as a flat tube support face 16, and a rectangular window 17 is provided at one open end of the passage 14 while a cylindrical fixing guide sleeve 18 at the other open end thereof. One or more rotation stopper projections 19 which are separated from each other in the circumferential direction of the guide sleeve 18 are formed on the front outer circumference of the guide sleeve 18.

The rotator 21 is provided with a rotary operation lever 21a and small- and large-diameter sections 22 and 23 supported rotatable in the bearing sections 12 and 13 of the holder 11. The rotator 21 also has a substantially chord-like tube closing section 24 between the supported sections 22 and 23 which presses the tube 1 passed through the passage 14 of the holder 11 against the tube support face 16 of the passage 14.

Further, the rotator 21 has tube opening and closing stopper recesses 25a and 25b on the outer circumference of the large-diameter supported section 23 thereof and the tube closing stopper recess 25b separated from the other one 25a in the circumferential surface of the section 23 extends all over the tube closing section 24 of the rotator 21.

When the rotator 21 is rotated relative to the holder 11 in the case of this clamp device 10,
1) the stopper projection 15 of the holder 11 is fitted into the stopper recess 25a to stop the rotator 21 at that position where the tube closing section 24 is kept inoperative, so that the tube 1 can be left open to allow fluid to flow through the tube 1 (see Figs. 5A and 5B), and
2) the stopper projection 15 of the holder 11 is fitted into the stopper recess 25b to stop the rotator 21 at that position where the tube closing section 24 is made operative, so that the tube 1 can be closed to stop the flow of fluid through the tube 1 (see Figs. 6A and 6B).

According to the clamp device 10, the supported sections 22 and 23 of the rotator 21 are positioned beside the passage 14 of the holder 11 and at that area where the passage 14 is projected. The center line passing through these supported sections 22 and 23 of the rotator 21 is positioned this time to cross the passage 14 in the inside of the passage 14.

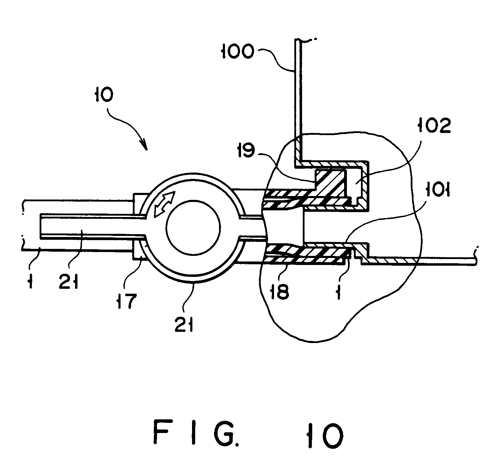

The fixing guide sleeve 18 and the rotation stopper projections 19 of the holder 11 are used as shown in Fig. 10 in the case of this clamp device 10. While holding the tube 1 in the guide sleeve 18, one end of the tube 1 passed through the passage 14 of the holder 11 and the front end of the guide sleeve 18 are fitted onto an outlet port 101 of a container 100 in which fluid such as blood and medicine is contained. This makes it unnecessary to hold or grip the holder 11 by hand and the rotary operation lever 21a can be thus rotated by fingers of one hand while keeping the holder 11 supported by the fluid container 100. No space is therefore needed around the clamp device 10 to allow the holder 11 to be held by hand, so that the clamp device 10 can be positioned nearer another equipment such as the container 100.

When one or more rotation stopper projections 19 on the front outer circumference of the guide sleeve 18 are engaged with recesses 102 around the outlet port 101 of the container 100, the holder 11 can be prevented from rotating round the tube 1.

When the stopper projection 15 of the holder 11 is switched from the tube opening stopper recess 25a to the tube closing stopper recess 25b of the rotator 21 to shift the rotator 21 from the tube opening to the tube closing position, while rotating the rotary operation lever 21a under such a state that the tube 1 is passed through the passage 14 of the holder 11, the tube closing plate 24 of the rotator 21 presses the tube 1 against the inner face 16 of the passage 14 to close the tube 1. The clamp device 10 can achieve the following merits 1) and 2) in this case, because the supported sections 22 and 23 of the rotator 21 are positioned beside the passage 14 of the holder 11 and at that area where the passage 14 is projected, as described above.
1) The whole of the clamp device 10 can be smaller-sized.
2) The pressing force gradually added to the surface of the tube 1 by the tube closing section 24 of the rotator 21 is stronger in a direction perpendicular to the axis of the tube 1. The tube 1 can be therefore more effectively closed as if it were pressedly cut by the tube closing section 24 in the direction perpendicular to its axis. As the result, it can be closed with a smaller force but with a higher reliability.
   In addition, the merits 1) and 2) can be realized with a higher reliability because the center line of the rotator 21 is positioned to cross the passage 14 in the inside of the passage 14.
   Further, the clamp device 10 can achieve the following merit because the holder 11 has the fixing guide sleeve 18.
3) When the fixing guide sleeve 18 of the holder 11 is fitted onto the outlet port 101 of the fluid container 100 together with the tube 1 passed through the passage 14 of the holder 11, it can be made unnecessary to hold or grip the holder 11 by hand. The rotary operation lever 21a can be thus rotated by fingers of one hand while keeping the holder 11 supported by the fluid container 100. Therefore, no space is needed around the clamp device 10 to allow its holder 11 to be held by hand, thereby enabling the clamp device 10 to be positioned nearer another equipment such as the fluid container 100.

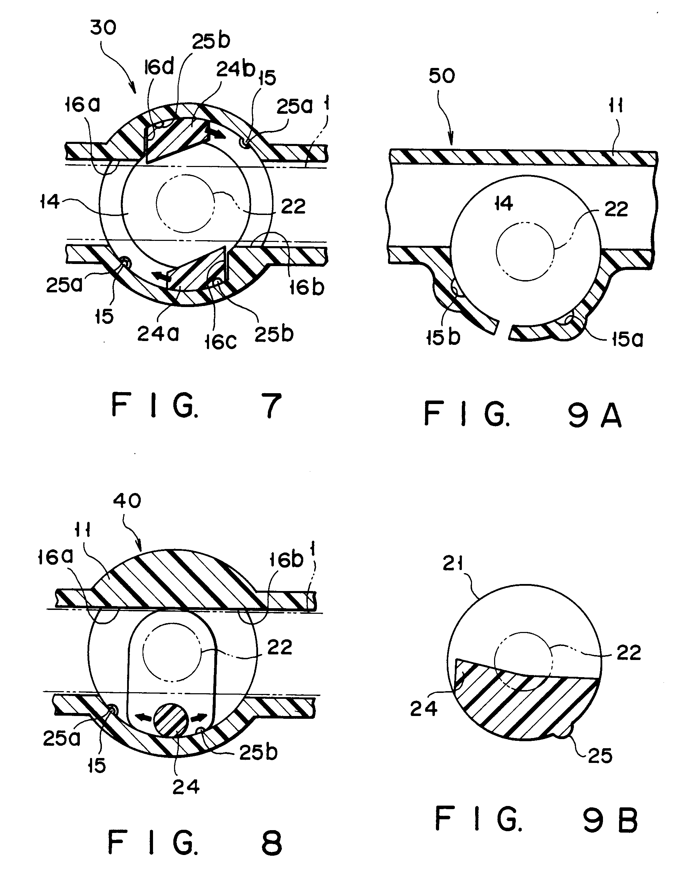

In the case of a second embodiment of the present invention shown in Fig. 7, a clamp device 30 is different from the above-described one 10 in that chord-like tube closing sections 24a and 24b are arranged between the small- and large-diameter supported sections 22 and 23 but at those two positions which are separated from each other by about 180° round the rotating axis of the rotator 21.

Those two inner faces of the passage 14 of the holder 11 which face the tube closing sections 24a and 24b of the rotator 21 serve as tube support faces 16a and 16b.

When the rotator 21 is switched from the opening position shown in Fig. 7 to the closing position in the case of the clamp device 30, therefore, both of the tube closing sections 24a and 24b press the tube 1 in the passage 14 against their corresponding tube support faces 16a and 16b, so that the tube 1 can be more reliably closed by the tube closing plates 24a and 24b at two positions in the axial direction of the tube 1. Reference numerals 16c and 16d in Fig. 7 denote stopper faces by which the rotator 21 is stopped at the tube opening position.

In the case of a third embodiment of the present invention shown in Fig. 8, a clamp device 40 is different from the above-described one 10 in that the tube closing section 24 of a rotator 31 is shaped like a column or rod.

The tube support faces 16a and 16b are formed on the inner face of the passage 14 of the holder 11 at two positions thereof so as to enable them to face the tube closing column 24 of the rotator 21 when the rotator 21 is rotated left or right from the tube opening position shown in Fig. 8.

When the rotator 21 is rotated left or right from the opening position shown in Fig. 8 to the closing position in the case of the clamp device 40, therefore, the tube closing column 24 presses the tube 1 in the passage 14 against the tube support face 16a or 16b to close the tube 1.

In the case of a fourth embodiment of the present invention shown in Figs. 9A and 9B, a clamp device 50 is different from the above-described one 10 in that the center line of the supported sections 22 and 23 of the rotator 21 is positioned to cross the passage 14 on the contour line of the passage 14.

Opening and closing stopper recesses 15a and 15b are formed on the inner circumferential surface of the holder 11 at two positions and the stopper projection 25 on outer circumferential surfaces of the large-diameter supported section 23 and the tube closing section 24 of the rotator 21 at a single position of the rotator 21. The rotator 21 is stopped at the opening position where the stopper projection 25 is fitted into the opening stopper recess 15a to make the tube closing section 24 inoperative or at the closing position where the stopper projection 25 is fitted into the closing stopper recess 15b to make the tube closing section 24 operative.

In the case of this clamp device 50, too, part of the supported sections 22 and 23 of the rotator 21 is positioned beside the passage 14 of the holder 11 and at that area where the passage 14 is projected. The whole of the clamp device 50 can be thus made more compact and the tube 1 can be closed with a smaller force but with a higher reliability.

It is preferable in the case of the present invention that the holder and the rotator are made of synthetic resin such as PP (polypropylene), AS (acrylonitrile-styrene), PS (polystyrene), ABS (acrylonitrile-butadiene-styrene), POM (polyoxymethylene), and PC (polycarbonate), excellent in rigidity, and particularly PC and ABS, more excellent in toughness and shock resistance, are more suitable. Or a filler may be blended with one of the above-mentioned matters to increase the strength of the holder and the rotator.

The following test was conducted using the clamp device 10. The pressure-tight and sealing capacity of the clamp device 10 was checked under such a condition that a PCV tube having an inner diameter of 6 mm and an outer diameter of 9 mm was set in the clamp device 10 and that air pressure was added to the tube through one end thereof while keeping the other end of the tube immersed in water. No air was leaked through the tube even when the air pressure added was increased to 0.05, 0.1, 0.15, 0.20, 0.26, 0.31 and 0.36 N (0.5, 1.0, 1.5, 2.0, 2.5., 3.0 and 3.5 Kgf/cm²). It was thus found that the clamp device 10 had a tube closing capacity which can resist the air pressure of 0.36 N (3.5 Kgf/cm²) added.

## Claims

1. A clamp device comprising;
a holder (11) having a passage (14) through which a tube (1) is passed in a first direction;
a rotator (21) attached to the holder (11) to be rotatable round its rotating axis which extends in a second direction;
bearing means (12, 13) formed on the inner face of the holder (11);
supported means (22, 23) formed on the rotator (21) and supported by the bearing means (12, 13) of the holder (11);
tube closing means (24, 24a, 24b) arranged at the rotator (21) and positioned eccentric to the rotating axis of the rotator (21), said tube closing means (24, 24a, 24b) being moved between a first position where the tube (1) is pressed-against the inner face of the passage (14) by the tube closing means and a second position where the tube (1) is released from the tube closing means when the rotator (21) is rotated;
a rotary operation lever (21a) attached to the rotator (21); and
stopper means (15, 15a, 15b, 25, 25a, 25b, 16c, 16d) for stopping the rotator (21) relative to the holder (11) at the first and second positions;
characterized in that the line extending along the rotating axis of the rotator (21) is positioned to cross the passage (14).

2. A clamp device according to claim 1, characterized in that said first and second directions are made substantially perpendicular to each other.

3. A clamp device according to claim 2, characterized in that the rotating axis of the rotator (21) is positioned to cross the passage (14).

4. A clamp device according to claim 3, characterized in that the rotating axis of the rotator (21) is positioned to cross the center line of the passage (14).

5. A clamp device according to claim 1, characterized in that said stopper means include first and second stopper members (25a, 25b) formed on the rotator (21) and a third stopper member (15) formed on the inner face of the holder (11) and the third stopper member (15) can be selectively engaged with the first and second stopper members.

6. A clamp device according to claim 5, characterized in that the first and second stopper members are recesses (25a, 25b) and the third stopper member is a projection (15).

7. A clamp device according to claim 1, characterized in that said stopper means include a first stopper member (25) formed on the rotator (21) and second and third stopper members (15a, 15b) formed on the inner face of the holder (11), and the first stopper member can be selectively engaged with the second and third stopper members.

8. A clamp device according to claim 7, characterized in that said first stopper member is a projection (25) and said second and third stopper members are recesses (15a, 15b).

9. A clamp device according to claim 1, characterized in that said tube closing means includes a single tube closing member (24).

10. A clamp device according to claim 1, characterized in that said tube closing means includes plural tube closing members (24a, 24b).

11. A clamp device according to claim 1, characterized in that said stopper means include members (16c, 16d) formed on the inner face of the holder (11) to engage with the tube closing means (24a, 24b) to stop the rotator (21) at the second position.

12. A clamp device according to claim 1, characterized in that said first position is formed plural relative to the second position in the both rotating directions of the rotator (21).

13. A clamp device according to claim 1, characterized in that a guide sleeve (18) by which part of the passage (14) is defined is formed on the holder (11), the tube (1) in the guide sleeve (18) is connected to a container (100), and the guide sleeve (18) includes a means (19) for fixing the clamp device to the container (100).

## Patentansprüche

1. Klemmvorrichtung, umfassend
- einen Halter (11), der eine Durchführung (14) aufweist, durch die ein Schlauch (1) in einer ersten Richtung durchgeführt wird;
- ein Drehteil (21), das so am Halter (11) angebracht ist, daß es um seine Drehachse drehbar ist, die sich in einer zweiten Richtung erstreckt;
- eine auf der Innenseite des Halters (11) gebildete Lagereinrichtung (12, 13);
- eine Trageinrichtung (22, 23), die auf dem Drehteil (21) gebildet und durch die Lagereinrichtung (12, 13) des Halters (11) getragen ist;
- eine Schlauchverschließeinrichtung (24, 24a, 24b), die am Drehteil (21) angeordnet ist und exzentrisch zur Drehachse des Drehteils (21) positioniert ist, wobei die Schlauchverschließeinrichtung (24, 24a, 24b) zwischen einer ersten Position, bei der der Schlauch (1) durch die Schlauchverschließeinrichtung gegen die Innenseite der Durchführung (14) gedrückt ist, und einer zweiten Position bewegt wird, bei der der Schlauch (1) von der Schlauchverschließeinrichtung freigegeben wird, wenn das Drehteil (21) gedreht wird;
- einen am Drehteil (21) angebrachten Drehbetätigungshebel (21a); und
- eine Anschlageinrichtung (15, 15a, 15b, 25, 25a, 25b, 16c, 16d) zum Anhalten des Drehteils (21) in bezug auf den Halter (11) bei der ersten und zweiten Position;
dadurch **gekennzeichnet,** daß die sich entlang der Drehachse des Drehteils (21) erstreckende Linie so positioniert ist, daß sie die Durchführung (14) kreuzt.

2. Klemmvorrichtung nach Anspruch 1, dadurch **gekennzeichnet,** daß die erste und zweite Richtung im wesentlichen senkrecht zueinander gemacht sind.

3. Klemmvorrichtung nach Anspruch 2, dadurch **gekennzeichnet,** daß die Drehachse des Drehteils (21) so positioniert ist, daß sie die Durchführung (14) kreuzt.

4. Klemmvorrichtung nach Anspruch 3, dadurch **gekennzeichnet,** daß die Drehachse des Drehteils (21) so positioniert ist, daß sie die Mittellinie der Durchführung (14) kreuzt.

5. Klemmvorrichtung nach Anspruch 1, dadurch **gekennzeichnet,** daß die Anschlageinrichtung am Drehteil (21) gebildete erste und zweite Anschlagelemente (25a, 25b) und ein an der Innenseite des Halters (11) gebildetes drittes Anschlagelement (15) aufweist und das dritte Anschlagelement (15) selektiv in Eingriff mit den ersten und zweiten Anschlagelementen gebracht werden kann.

6. Klemmvorrichtung nach Anspruch 5, dadurch **gekennzeichnet,** daß die ersten und zweiten Anschlagelemente Aussparungen (25a, 25b) sind und das dritte Anschlagelement ein Vorsprung (15) ist.

7. Klemmvorrichtung nach Anspruch 1, dadurch **gekennzeichnet,** daß die Anschlageinrichtungen ein am Drehteil (21) gebildetes erstes Anschlagelement (25) und an der Innenseite des Halters (11) gebildete zweite und dritte Anschlagelemente (15a, 15b) umfassen und das erste Anschlagelement selektiv mit den zweiten und dritten Anschlagelementen in Eingriff gebracht werden kann.

8. Klemmvorrichtung nach Anspruch 7, dadurch **gekennzeichnet,** daß das erste Anschlagelement ein Vorsprung (25) ist und die zweiten und dritten Anschlagelemente Aussparungen (15a, 15b) sind.

9. Klemmvorrichtung nach Anspruch 1, dadurch **gekennzeichnet,** daß die Schlauchverschließeinrichtung ein einzelnes Schlauchverschließelement (24) umfaßt.

10. Klemmvorrichtung nach Anspruch 1, dadurch **gekennzeichnet,** daß die Schlauchverschließeinrichtung mehrere Schlauchverschließelemente (24a, 24b) umfaßt.

11. Klemmvorrichtung nach Anspruch 1, dadurch **gekennzeichnet,** daß die Anschlageinrichtungen an der Innenseite des Halters (11) gebildete Elemente (16c, 16d) zum Eingreifen mit der Schlauchverschließeinrichtung (24a, 24b) umfassen, um das Drehteil (21) bei der zweiten Position zu stoppen.

12. Klemmvorrichtung nach Anspruch 1, dadurch **gekennzeichnet,** daß die erste Position mehrfach in bezug auf die zweite Position in beiden Drehrichtungen des Drehteils (21) gebildet ist.

13. Klemmvorrichtung nach Anspruch 1, dadurch **gekennzeichnet,** daß am Halter (11) eine Führungshülse (18) gebildet ist, durch die ein Teil der Durchführung (14) abgegrenzt ist, der Schlauch (1) in der Führungshülse (18) mit einem Behälter (100) verbunden ist und die Führungshülse (18) eine Einrichtung (19) zur Befestigung der Klemmvorrichtung am Behälter (100) umfaßt.

## Revendications

1. Dispositif de pinçage comprenant :
un support (11) comportant un passage (14) à travers lequel on fait passer un tube (1) dans une première direction;
un tournant (21) fixé au support (11) pour être entraîné en rotation autour de son axe de rotation qui s'étend dans une seconde direction;
des moyens (12, 13) formant palier formés sur la face intérieure du support (11);
des moyens supportés (22, 23) formés sur le tournant (21) et supportés par les moyens 12, 13) formant palier du support (11),
des moyens (24, 24a, 24b) de fermeture de tube disposés à l'endroit du tournant (21) et positionnés de façon excentrée par rapport à l'axe de rotation du tournant (21), lesdits moyens (24, 24a, 24b) de fermeture de tube étant déplacés entre une première position où le tube (1) est pressé contre la face intérieure du passage (14) par le moyen de fermeture de tube et une seconde position où le tube (1) est libéré des moyens de fermeture de tube lorsque l'on fait tourner le tournant (21);
un levier de manoeuvre rotatif (21 a) fixé au tournant (21), et des moyens d'arrêt (15, 15a, 15b, 25, 25a, 25b, 16c, 16d) pour arrêter le tournant (21) par rapport au support (11) dans les première et seconde positions;
caractérisé en ce que la ligne s'étendant le long de l'axe de rotation du tournant (21) est positionnée de manière à couper le passage (14).

2. Dispositif de pinçage selon la revendication 1, caractérisé en ce que lesdites première et seconde directions sont sensiblement perpendiculaires l'une à l'autre.

3. Dispositif de pinçage selon la revendication 2, caractérisé en ce que l'axe de rotation du tournant (21) est positionné de manière à couper le passage (14).

4. Dispositif de passage selon la revendication 3, caractérisé en ce que l'axe de rotation du tournant (21) est positionné de manière à couper l'axe du passage (14).

5. Dispositif de pinçage selon la revendication 1, caractérisé en ce que lesdits moyens d'arrêt comprennent des premier et second éléments d'arrêt (25a, 25b) formés sur le tournant (21) et un troisième élément d'arrêt (15) formé sur la face intérieure du support (11) et le troisième élément d'arrêt (15) peut être amené sélectivement contre les premier et second éléments d'arrêt.

6. Dispositif de pinçage selon la revendication 5, caractérisé en ce que les premier et second éléments d'arrêt sont des évidements (25a, 25b) et le troisième élément d'arrêt est une saillie (15).

7. Dispositif de pinçage selon la revendication 1, caractérisé en ce que lesdits moyens d'arrêt comprennent un premier élément d'arrêt (25) formé sur le tournant (21) et des second et troisième éléments d'arrêt (15a, 15b) formés sur la face intérieure du support (11), et le premier élément d'arrêt peut être amené sélectivement contre les seconds et troisième éléments d'arrêt.

8. Dispositif de pinçage selon la revendication 7, caractérisé en ce que ledit premier élément d'arrêt est une saillie (25) et lesdits second et troisième éléments d'arrêt sont des évidements (15a, 15b).

9. Dispositif de pinçage selon la revendication 1, caractérisé en ce que lesdits moyens de fermeture de tube comprennent un seul élément (24) de fermeture de tube.

10. Dispositif de pinçage selon la revendication 1, caractérisé en ce que lesdits moyens de fermeture de tube comprennent plusieurs éléments (24a, 24b) de fermeture de tube.

11. Dispositif de passage selon la revendication 1, caractérisé en ce que lesdits moyens d'arrêt comprennent des éléments (16c, 16d) formés sur la face intérieure du support (11) de manière à porter contre les moyens (24a, 24b) de fermeture de tube pour arrêter le tournant (21) dans la seconde position.

12. Dispositif de passage selon la revendication 1, caractérisé en ce que que ladite première position est formée de façon multiple par rapport à la seconde position dans les deux directions de rotation du tournant (21).

13. Dispositif de passage selon la revendication 1, caractérisé en ce qu'un manchon de guidage (18) au moyen duquel une partie du passage (14) est définie est formé sur le support (11), le tube (1) dans le manchon de guidage (18) est raccordé à un récipient (100), et le manchon de guidage (18) comprend un moyen (19) pour fixer le dispositif de passage au récipient (100).
